Europäisches Patentamt

European Patent Office

Office européen des brevets

(11) Publication number: 0 424 930 B1

# EUROPEAN PATENT SPECIFICATION

(45) Date of publication of patent specification: 19.07.95

(51) Int. Cl.⁶: $C12N\ 5/00$, C12N 5/04

(21) Application number: 90120442.0

(22) Date of filing: 25.10.90

The file contains technical information submitted after the application was filed and not included in this specification

(54) Method for culturing tomato protoplasts.

(30) Priority: 27.10.89 JP 280601/89

(43) Date of publication of application:
02.05.91 Bulletin 91/18

(45) Publication of the grant of the patent:
19.07.95 Bulletin 95/29

(84) Designated Contracting States:
AT BE CH DE DK ES FR GB GR IT LI LU NL SE

(56) References cited:
EP-A- 0 129 668
JP-A- 6 255 077

J. PLANT PHYSIOL., vol. 132, 1988, pages
552-556, Gustav Fischer Verlag,Stuttgart, DE;
K.I. DRAGET et al.: "Regeneration, cultivation
anddifferentiation of plant protoplasts immo-
bilized in Ca-alginate beads"

CHEMICAL ABSTRACTS, vol. 100, 1984, page
225, abstract no. 31831j, Columbus,Ohio, US;
R.D. SHILLITO et al.: "Agarose plating and a
bead type culturetechnique enable and
stimulate development of protoplast-derived
colonies in a number of plant species" &
PLANTCELL REP. 1983, 2(5), 244-7

(73) Proprietor: Sakata Seed Corporation
7-1, Nakamachidai 2-chome,
Tsuzuki-ku
Yokohama 224 (JP)

(72) Inventor: Komiya, Takeya
7-43, Mianmisakurazuka 1-chome
Toyonaka,
Osaka 560 (JP)
Inventor: Ozaki, Kazuo
4-104, 1-14, Koaza-saihoji,
Aza-enmyoji
Ohyamazaki-cho,
Otokuni-gun,
Kyoto 618 (JP)
Inventor: Uno, Yukari
42-201, 1-23, Takanohigashihiraki-cho
Sakyo-ku,
Kyoto 606 (JP)

(74) Representative: von Kreisler, Alek,
Dipl.-Chem. et al
Patentanwälte
von Kreisler-Selting-Werner
Postfach 10 22 41
D-50462 Köln (DE)

CHEMICAL ABSTRACTS, vol. 96, 1982, page 369, abstract no. 213784c, Columbus,Ohio, US; E.N. MBANASO et al.: "Alginate: an alternative to agar in plantprotoplast culture", & PLANT SCI. LETT. 1982,25(1), 61-6

BIOLOGICAL ABSTRACTS, vol. 83, 1987, abstract no. 37815, Philadelphia, PA, US;D.M. TRICOLI et al.: "Culture of soybean (Glycine max) mesophyll protoplasts inalginate beads", & PLANT CELL. REP. 5(5): 334-337,1986

Plant Cell Reports (1985) Vol. 4, pp. 23-27

JPA-62 55077

**Description**

The present invention relates to a method for culturing tomato protoplasts.

Protoplast culture is a technique essential to plant breeding based on cell fusion, gene recombination or somaclonal variation.

There have been many studies on culturing tomato protoplast, most of which deal with the wild species (*Lycopersicon peruvianum*). Little success has been achieved in tomato protoplast culture with the cultivated species (*L. esculentum*), whose protoplasts are difficult to culture [Hikoyuki Yamaguchi ed.: Syokubutsu Idenshi Sosa Gijyutsu, p. 195 (1985)]. Accordingly, it is desired that a new technique for culturing protoplast, that is easily applicable to cultivated varieties of tomato, be developed to permit utilization of cultivated tomato protoplasts as a means of tomato breeding.

In general, liquid medium has been used to culture tomato protoplasts [E. A. Shahin: Cell Culture and Somatic Cell Genetics of Plants, vol. *1*, p. 370 (1984)]. Also, Morgan et al. reported a method of culturing tomato protoplast in which tomato protoplasts were embedded in agar medium [A. Morgan, E. C. Cocking: Z. Pflanzenphysiol., *106*, 97 (1982)].

However, even when these methods are applied to ordinary cultivated varieties of tomato, the obtained frequencies of cell division are low, and often colony formation does not occur. As well, for the confirmation of introduction of gene causing a drug resistance into plant cell, protoplasts should be isolated and its resistance should be checked according to the conventional method. However, when protoplasts are cultured in a liquid medium, callus collapses, and therefore each callus is quite difficult to be cloned. Collapse of callus must be prevented by the use of solid medium, but the method necessitates heating, which exerts an adverse effect such as causing death of cell on cells.

Also, methods for culturing protoplasts of plants (soybeans, tobaccos, petunias) using alginic acid in solid medium are disclosed in Japanese Patent Publication Open to Public Inspection No. 55077/1987 and Plant Science Letters, *25*, p. 61-66 (1982), but colony formation ratios obtained using alginic acid are lower than those obtained using agar. It has not been recognized that use of alginic acid for plant protoplast culture is advantageous.

The present inventors have quite unexpectedly found that the use of alginic acid as a medium solidifying agent in a liquid medium for culturing tomato protoplast offers remarkable improvement in cell division ratio and colony formation ratio. The present inventors made further investigations based on this unexpected finding, and developed the present invention.

Accordingly, the present invention relates to:

(1) a method for culturing tomato protoplasts characterized in which tomato protoplasts are embedded in solid medium containing alginic acid or its salts and cultured in the presence of a liquid medium added thereto, wherein said liquid medium is added so that part of the solid medium is kept in contact with air, and

(2) tomato protoplasts embedded in a solid medium containing alginic acid or its salts as defined above. The method for culturing protoplast according to the present invention as carried out usually in a following way of the four consecutive processes.

1) a process of isolating tomato protoplasts (protoplast isolation process),
2) a process of embedding the protoplasts in alginic acid (protoplast embedding process),
3) a process of culturing the protoplasts (protoplast culture process) and
4) a process of regenerating plant bodies (plant body regeneration process).

The protoplast culture method of the present invention is hereinafter described in the order of the four processes.

1) Protoplast isolation process

Protoplasts of tomato can easily be obtained from organs and cultured cells of tomato by a method known per se. Specifically, pectinase (i.e. an enzyme that lyzes pectin, which is an intercellular substance), cellulase (i.e. an enzyme that lyzes the cell wall), an osmoticum such as mannitol, sorbitol, and glucose, as well as a known plant tissue medium and a conventional plant hormone, are dissolved in a CPW solution (Table 1) or MES buffer as appropriate. The resulting enzyme solution is adjusted to a pH of 5.0 to 6.5. In this enzyme solution, the starting material, i.e. organs or cells of tomato is kept standing or shaken at 20 to 35°C for 5 to 24 hours and then thus produced protoplasts are isolated as follows. After above enzyme treatment, the sample solution may be filtered through a 50 to 100 $\mu$m mesh to remove the undigested cell mass and subjected to centrifugation or density gradient treatment with sucrose, or Ficoll according to the per se known method to separate the enzyme solution from the tomato protoplasts, which are then washed with, for example, a CPW solution containing an osmoticum (hereinafter referred to as CPW solution) to yield purified protoplasts.

Table 1

| Composition of CPW | |
|---|---|
| Component | Concentration (mg/ℓ) |
| $KH_2PO_4$ | 25.9 |
| $KNO_3$ | 101.1 |
| $CaCl_2 \cdot 2H_2O$ | 1484.9 |
| $MgSO_4 \cdot 7H_2O$ | 246.5 |
| KI | 0.159 |
| $CuSO_4 \cdot 5H_2O$ | 0.025 |

2) Protoplast embedding process

The protoplasts obtained by isolation and purification after enzyme treatment as explained above are washed with a CPW solution free of calcium chloride, followed by filtration or centrifugation. The collected protoplasts, optionally after suspending them in a liquid medium, are mixed uniformly with a plant tissue culture medium (hereinafter referred to as medium) containing sodium alginate (0.5 to 5.0%). Then, using a Komagome pipette, this mixture is added dropwise to an aqueous solution of calcium chloride (about 20 to 100 mM) containing an osmoticum and a medium. The mixture is kept standing for 30 minutes to 24 hours, and then gelated solid body in which tomato protoplasts are embedded in solid medium are formed in the calcium chloride solution.

Sodium alginate may be replaced with arginic acid or another salt thereof (e.g. potassium alginate, ammonium alginate). In this invention there may be used one or more than two selected from alginic acid and its salts. Preference is given to sodium alginate. Examples of osmoticum include sucrose, mannitol, glucose and sorbitol, with preference given to sucrose alone or in combination with one or more other osmoticum at 0.2 to 0.7 M. The mixture is thoroughly washed with a CPW solution to finally obtain tomato protoplasts, embedded in a normally beady solid medium containing alginic acid or its salt.

Examples of usable media (i.e. the above-mentioned culture media) include Murashige-Skoog medium (hereinafter referred to as MS), Gamborg B5 medium (hereinafter referred to as B5), Sapata's medium, Shahin's medium and modifications thereof. However, these media are used after the calcium chloride contained therein is removed.

These media may be formulated with carbon sources, nitrogen sources, inorganic salts, organic substances etc. as desired.

Examples of carbon sources include sugars such as sucrose, glucose, fructose and maltose, as well as soluble starch. Examples of nitrogen sources include nitrates and ammonium salts, but it is preferable to use no ammonium salt or only a small amount of ammonium salt. Examples of inorganic salts include those containing an element such as phosphorus, potassium, calcium, magnesium, manganese, copper, zinc, molybdenum, boron, iron, cobalt or nickel. Examples of organic substances include vitamins such as inositol, nicotinic acid, pyridoxine hydrochloride, thiamin hydrochloride, calcium pantothenate, folic acid, p-aminobenzoic acid, biotin, choline chloride, riboflavin, ascorbic acid, vitamin A, vitamin $D_3$ and vitamin $B_{12}$; organic acids such as sodium pyruvate, citric acid, malic acid and fumaric acid; and natural substances such as coconut milk, casein hydrolyzates and yeast extracts. Particularly for the good formation of protoplast colonies, the addition of inositol is preferred, preferably at a ratio of 0.1 to 8.0 g/ℓ.

The medium may also be supplemented with plant growth regulators, such as auxins and cytokinins, as desired. Examples of such auxins include 2,4-dichlorophenoxyacetic acid (hereinafter referred to as 2,4-D), 2,4-dichlorophenylacetic acid, indole-3-acetic acid (hereinafter referred to as IAA), indole-3-butyric acid (hereinafter referred to as IBA), 1-naphthaleneacetic acid (hereinafter referred to as NAA), 2-naphthoxyacetic acid, parachlorophenoxyacetic acid, 2,4,5-trichlorophenoxyacetic acid and 1-naphthaleneacetamide. Examples of usable cytokinins include 6-benzyladenine (hereinafter referred to as BAP), 2-isopentyladenine, 2-isopentenyladenine, kinetin, zeatin, dihydrozeatin, zeatin riboside and diphenylurea. Particularly, 2,4-D, NAA,

4

of the auxins and BAP of the cytokinins are often used.

Auxins are normally added to the medium in a ratio of 0.01 to 20 ppm. Cytokinins are normally added in a ratio of 0.01 to 15 ppm.

Of course, organic or inorganic acids, alkalis, buffers, are added to adjust the pH of the medium to 5.0 to 8.0.

The dropwise addition of alginic acid or its salts to a solution of calcium chloride forms a gelated solid body embedding tomato protoplasts (hereinafter referred to as bead) and facilitates culture medium renewal.

The present method using beads-shaped gelated solid body is advantageous in that protoplast growth is not adversely affected by mechanical impact or by heating, as seen in agarose embedding methods, and is free of shortcomings as found in the liquid culture method, such as the growth inhibitory action caused by wastes excreted from cultured cells and the division or survival inhibition phenomenon due to protoplast coagulation.

## 3) Protoplast culture process

Protoplasts are cultured as follows: The protoplast-embedding alginic acid bead which is usually shaped in beads is transferred to a container, such as a plastic petri dish having a diameter of 3 to 30 cm, or an Erlenmeyer flask having a capacity of 20 to 1,000 mℓ, containing 1 to 300 mℓ, preferably 4 to 20 mℓ, of a liquid culture medium containing carbon sources, plant hormones, as described above (however containing calcium chloride), and is kept standing or shaken. The amount of the culture medium is preferable adjusted so that part of the beads are kept in contact with air. Any known liquid culture medium for plant protoplast culture can be used.

Although culture conditions essentially vary depending upon the medium's status and composition, culture method and other aspects, it is desirable that protoplasts be cultured in the dark initially and that lighting be intensified gradually with protoplast growth. Culture is carried out at 15 to 35°C, preferably 25 to 30°C.

The liquid culture medium is renewed every 3 to 14 days, preferably every 5 to 10 days. Concerning the adjustment of osmotic pressure in the culture medium, it is desirable that the concentration of, for example, sucrose, mannitol or glucose, be reduced or increased as appropriate, preferably at a rate of 0.01 to 0.1 M every 5 to 10 days.

After the above-mentioned culture tomato protoplast-derived calli are formed for the purpose of plant body regeneration.

## 4) Plant body regeneration process

The calli obtained by protoplast culture are transferred to a medium that permits shoot regeneration, such as B5 medium or MS medium supplemented with sucrose, glucose or another carbon source at 0.3 to 10.0%. Auxins such as NAA, 2,4-D, IAA and IBA, normally at 0 to 10 ppm, and cytokinins such as BAP, kinetin and zeatin, normally at 0 to 20 ppm, may be added as plant hormone. Also a medium solidifying agent such as agar, agarose or Gelrite may also be added. Use of this medium permits shoot regeneration and rooting to produce plant bodies of tomato. In addition to the medium described above, other media known to induce shoots from plant calli can be used as desired.

Culturing is usually carried out under artificial lighting of 1,000 to 20,000 lux, preferably 3,000 to 10,000 lux, at 15 to 35°C, preferably 25 to 30°C. After the culturing for 2 to 4 weeks the culture medium is preferably replaced by a new culture medium. That is to say, it is preferable that subculture interval be 2 to 4 weeks. Sub-culture is repeated until the regeneration of protoplants is accomplished.

Complete plants are thus regenerated from protoplasts in 10 days to 1 year.

Further, tomato plants resistant to harmful plant viruses, herbicides, and insects, can be produced in an industrial scale by (1) introducing for example, gene coding for the coat protein of plant viruses, such as cucumber mosaic virus (CMV), tobacco mosaic virus (TMV), etc. into the protoplasts in advance to the culturing process of the protoplasts according to the present invention, (2) culturing to the thus produced protoplasts according to the present invention and (3) regenerating the thus cultured protoplasts into plant bodies.

The genes employed in the above step (1) give plants characteristics of resistance to virus, herbicides or insects.

The present invention is hereinafter described in more detail by means of the following examples.

[Example]

1) Protoplast isolation process

Seeds of the subject plant tomatoes [variety: Paresu (Takii & Company) and Baby Tomato (Yamato Noen)] were sterilized by sequential immersion in 70% ethanol for 1 minute and a 1% sodium hypochlorite solution for 10 minutes and then thrice washed with sterile water, after which they were sown on the MS agar medium shown in Table 2 (3% sucrose, 0.8% agar) and allowed to germinate under artificial lighting (white fluorescent lamp, 2,000 lux, 16 hr daylength) at 25°C.

After an enzyme solution (0.3% Macerozyme R-10, 1.0% Cellulase Onozuka R-10, 0.4 M mannitol, CPW solution, pH 5.8) was added in an amount of 20 ml per gram cotyledons of seedlings (1 week after sowing), the cotyledons were shredded and kept standing for enzyme treatment at 25 to 30°C in the dark for 16 hours.

After completion of the treatment, the protoplast suspension was filtered through 100 and 50 $\mu$m nylon meshes to remove the undigested cell mass, followed by centrifugation (70xg, 5 minutes) and density gradient elution with a 20% sucrose solution. The resulting eluate was twice washed with a CPW culture containing 0.4 M mannitol (hereinafter referred to as washing solution) to yield purified protoplasts.

2) Protoplast embedding process

The purified protoplasts thus obtained were once again washed with a washing solution free of calcium chloride and then mixed uniformly with the protoplast culture medium (OFK-1 medium) containing 0.75% sodium alginate (produced by Wako Pure Chemical Industries Ltd.), shown in Table 3. After adjustment to a density of 4 to 9 x $10^4$ protoplasts/ml, this mixture was added dropwise, using a sterile pipette, to 10 ml of a 50 mM calcium chloride solution (containing OFK-1 medium) in a sterile plastic petri dish having a diameter of 9 cm. This mixture was kept standing at 25°C for 6 hours to yield a globular solid having a diameter of about 5 mm (hereinafter referred to as bead).

3) Protoplast culture process

After this bead was twice washed with a washing solution, the OFK-2 medium (4 ml) shown in Table 3 was added, followed by standing culture at 27.5°C in the dark.

Seven days after initiation of culture, the medium was replaced with another OFK-2 medium (4 ml) having the same composition as above, except that the sucrose density alone was adjusted to 0.2 M, followed by shaking culture (40 rpm) at 25°C in the dark. Colony (8 or more cells) formation ratio is shown in Table 4.

4) Plant body regeneration process

Fourteen days after initiation of culture, the bead was placed on a shoot regeneration medium prepared by solidifying a mixture of the MS medium shown in Table 2 and 0.2 mg/l IAA, 2.0 mg/l zeatin and 5% sucrose by the addition of 0.3% Gel-lite (gellan gum, produced by Kelco Co.), followed by culturing at 25°C under artificial lighting (white fluorescent lamp, about 3,000 lux, 16 hr daylength).

Thirty days after initiation of culture, the calli grown in the bead were transferred to another shoot regeneration medium having the same composition as above except that the sucrose density alone was changed to 2%, followed by culturing under the same conditions as above. Shoots regenerated on the upper portion of the calli 45 days after initiation of culture.

After being grown further, these shoots were transplanted to root-inducing medium (MS medium, 0.01 mg/l IBA, 1% sucrose, 1% agar, pH 5.8) and cultured under the same conditions as above. As a result, roots were induced and complete plants regenerated. The redifferentiation ratio is shown in Table 5. Control experiments were conducted by protoplast culture using agar in place of alginic acid (agarose method) and by culturing purified protoplasts on OFK-2 medium (liquid method).

Table 2

| Murashige-Skoog (MS) Medium | |
|---|---|
| Component | Concentration (mg/ℓ) |
| $KNO_3$ | 1,900 |
| $NH_4NO_3$ | 1,650 |
| $KH_2PO_4$ | 170 |
| $CaCl_2 \cdot 2H_2O$ | 440 |
| $MgSO_4 \cdot 7H_2O$ | 370 |
| $FeSO_4 \cdot 7H_2O$ | 27.8 |
| $Na_2$-EDTA | 37.3 |
| $MnSO_4 \cdot 4H_2O$ | 22.3 |
| $ZnSO_4 \cdot 7H_2O$ | 8.6 |
| $CuSO_4 \cdot 5H_2O$ | 0.025 |
| $CoCl_2 \cdot 6H_2O$ | 0.025 |
| KI | 0.83 |
| $H_3BO_3$ | 6.2 |
| $Na_2M_0O_4 \cdot 2H_2O$ | 0.25 |
| Myoinositol | 100 |
| Nicotinic acid | 0.5 |
| Pyridoxine hydrochloride | 0.5 |
| Thiamin hydrochloride | 0.1 ~ 1.0 |
| Glycine | 2 |
| pH | 5.7 ~ 5.8 |

EP 0 424 930 B1

Table 3  Protoplast Culture Medium

| Component | OFK-1 (mg/$\ell$) | OFK-2 (mg/$\ell$) |
|---|---|---|
| $KNO_3$ | 1900 | 1900 |
| $CaC\ell_2 \cdot 2H_2O$ | – | 440 |
| $MgSO_4 \cdot 7H_2O$ | 370 | 370 |
| $KH_2PO_4$ | 170 | 170 |
| $FeSO_4 \cdot 7H_2O$ | 27.8 | 27.8 |
| $Na_2$-EDTA | 37.3 | 37.3 |
| $MnSO_4 \cdot 4H_2O$ | 22.3 | 22.3 |
| $ZnSO_4 \cdot 7H_2O$ | 8.6 | 8.6 |
| $CuSO_4 \cdot 5H_2O$ | 0.025 | 0.025 |
| $CoC\ell_2 \cdot 6H_2O$ | 0.025 | 0.025 |
| KI | 0.83 | 0.83 |
| $H_3BO_3$ | 6.2 | 6.2 |
| $Na_2M_0O_4 \cdot 2H_2O$ | 0.25 | 0.25 |
| Nicotinic acid | 2.5 | 2.5 |
| Thiamin hydrochloride | 10 | 10 |
| Pyridoxine hydrochloride | 1 | 1 |
| Folic acid | 0.5 | 0.5 |
| Biotin | 0.05 | 0.05 |
| Calcium D-pantothenate | 0.5 | 0.5 |
| Choline chloride | 0.1 | 0.1 |
| Glycine | 0.5 | 0.5 |
| Caseine hydrolyzate | 150 | 150 |
| L-cysteine | 1 | 1 |
| Malic acid | 10 | 10 |
| Ascorbic acid | 0.5 | 0.5 |

8

| Component | OFK-1 (mg/ℓ) | OFK-2 (mg/ℓ) |
|---|---|---|
| Adenine sulfate | 40 | 40 |
| L-glutamine | 100 | 100 |
| Myoinositol | 4,600 | 4,600 |
| Riboflavin | 0.25 | 0.25 |
| Sucrose | 102,700 (0.3 M) | 85,600 (0.25 M) |
| NAA | 0.5 | 0.5 |
| 2,4-D | 1.0 | 1.0 |
| BAP | 0.5 | 0.5 |
| pH | 5.7 ~ 5.8 | 5.7 ~ 5.8 |

Table 4

| Colony Formation Ratio (%)* | | | |
|---|---|---|---|
| Duration of culture | Method of culture | | |
| | Agarose method | Liquid method | Alginic acid bead method |
| 14 days | 0 | 0 | 63.6 |
| 30 days | 3.1 | 22.6 | / Note) |

* = (colony count/number of subject protoplasts) x 100
Note: Colony formation ratio was not determined, since the procedure was already shifted to the next process (plant regeneration process).

Table 5

| Redifferentiation Ratio (%)* | | |
|---|---|---|
| Duration of culture | Method of culture | |
| | Liquid method | Alginic acid bead method |
| 80 days | 0 | 13.2% |

* = (number of redifferentiated calli/number of cultured calli) x 100

From Tables 4 and 5, it is evident that the tomato protoplast culture method of the present invention offers significantly higher values of colony formation ratio and redifferentiation ratio, in comparison with conventional methods.

9

**Claims**

1. A method for culturing tomato protoplasts characterized in which tomato protoplasts are embedded in a solid medium containing alginic acid or its salts and cultured in the presence of a liquid medium added thereto, wherein said liquid medium is added so that part of the solid medium is kept in contact with air.

2. Tomato protoplasts embedded in a solid medium containing alginic acid or its salts according to claim 1.

**Patentansprüche**

1. Verfahren zum Kultivieren von Tomatenprotoplasten, dadurch gekennzeichnet, daß Tomatenprotoplasten in ein festes Medium, das Alginsäure oder ihre Salze enthält, eingebettet und in Gegenwart eines zugesetzten flüssigen Mediums kultiviert werden, wobei das flüssige Medium so zugesetzt wird, daß ein Teil des festen Mediums in Kontakt mit Luft gehalten wird.

2. Tomatenprotoplasten, die in ein festes Medium gemäß Anspruch 1, das Alginsäure oder ihre Salze enthält, eingebettet sind.

**Revendications**

1. Procédé de culture de protoplastes de tomate, caractérisé en ce qu'on incorpore des protoplastes de tomate dans un milieu solide contenant de l'acide alginique ou de ses sels, et on les cultive en présence d'un milieu liquide qu'on y a ajouté, ce milieu liquide étant ajouté de telle façon qu'une partie du milieu solide reste en contact avec l'air.

2. Protoplastes de tomate incorporés dans un milieu solide contenant de l'acide alginique ou de ses sels, conformément à la revendication 1.